# FASCICULE DE BREVET EUROPEEN

(11) **EP 0 969 850 B1**
(45) Date de publication et mention de la délivrance du brevet: **20.06.2001**
(21) Numéro de dépôt: 98910822.0
(22) Date de dépôt: 24.02.1998
(51) Int. Cl.: A61K 35/52, A01N 1/02

(54) **DILUEUR DE SPERME COMPRENANT DU PHOSPHOCASEINATE NATIF OU DE LA BETA-LACTOGLOBULINE, SON PROCEDE DE PREPARATION ET SES UTILISATIONS**
SPERMAVERDÜNNER DER NATIVES PHOSPHOCASEINAT ODER BETA LACTOGLOBULIN ENTHÄLT, VERFAHREN ZU SEINER HERSTELLUNG UND SEINE VERWENDUNGEN
SPERM EXTENDER COMPRISING NATIVE PHOSPHOCASEINATE OR BETA-LACTOGLOBULIN, METHOD FOR PREPARING SAME AND USES

(30) Priorité: 25.02.1997 FR 9702198
(43) Date de publication de la demande: 12.01.2000
(73) Titulaire: INSTITUT NATIONAL DE LA RECHERCHE AGRONOMIQUE, F-75341 Paris Cédex 07 (FR)
(72) Inventeur: BATELLIER, Florence, F-37000 Tours (FR); MAGISTRINI, Michèle, F-37550 Saint-Avertin (FR); MAUBOIS, Jean-Louis, F-35740 Pace (FR); PALMER, Eric, F-78720 Maincourt (FR)
(74) Mandataire: Vialle-Presles, Marie José
(86) Numéro de dépôt international: FR9800361
(87) Numéro de publication internationale: WO9837904

(56) Documents cités:
- FR-A- 1 043 684
- GB-A- 2 057 247
- DATABASE WPI Section Ch, Week 8707 Derwent Publications Ltd., London, GB; Class B04, AN 87-046449 XP002045866 & JP 62 003 785 A (KUMABE K) , 9 janvier 1987
- JASKO D J ET AL: "EFFECT OF SEMINAL PLASMA DILUTION OR REMOVAL ON SPERMATOZOAL MOTION CHARACTERISTICS OF COOLED STALLION SEMEN." THERIOGENOLOGY 35 (6). 1991. 1059-1068, XP002069670
- BATELLIER F ET AL: "Effect of milk fractions on survival of equine spermatozoa." THERIOGENOLOGY 48 (3). 1997. 391-401, XP002069671

## Description

La présente Invention est relative à l'utilisation de fractions protéiques laitières purifiées pour améliorer la survie des spermatozoïdes et leur aptitude à la fécondation, en vue de l'insémination artificielle ou de toute autre pratique de reproduction assistée.

Les techniques d'insémination artificielle qui sont de plus en plus utilisées actuellement, posent des problèmes de conservation du sperme en attente de l'insémination.

En effet, après la récolte, le sperme perd rapidement son pouvoir fécondant. Pour remédier à cette situation, et permettre la conservation du sperme jusqu'à son utilisation pour l'insémination artificielle, on utilise habituellement un milieu dénommé "dilueur" dans lequel on dilue le sperme aussitôt après sa récolte.

La durée de conservation que l'on peut obtenir en présence de dilueur dépend de l'espèce concernée et des conditions de conservation, et en particulier du type de dilueur choisi ; elle varie en général de quelques heures à quelques jours. Chez certaines espèces, on peut prolonger cette durée par la congélation du sperme ; cependant, cette technique entraîne également la destruction ou l'inactivation d'un nombre important de spermatozoïdes, et donc une baisse de fertilité du sperme.

L'obtention de dilueurs permettant d'améliorer la conservation et la capacité fécondante des spermatozoïdes a fait l'objet de diverses recherches.

Parmi les dilueurs les plus fréquemment préconisés pour la conservation du sperme, on citera en particulier le lait, et les dilueurs à base de lait, qui sont utilisés par exemple chez les espèces caprine, ovine, bovine, et équine.

Par exemple, chez cette dernière espèce, le dilueur dénommé « INRA 82 », décrit par PALMER [10^{th} Int. Congress on Anim. Reprod. and AI, Urbana-Champaign, IL USA, 3:377, (1984)] ou le dilueur de KENNEY [KENNEY et al., Proc. 21^{st} Am. Assoc. Equine Pract., 327-336, (1975)] sont actuellement les plus utilisés pour la semence d'étalon en vue d'inséminations artificielles immédiates ou différées.

La température de conservation joue également un rôle important, bien que les différentes observations réalisées à ce sujet, en utilisant des dilueurs différents, aboutissent à des résultats divergents. Par exemple, chez le cheval, VARNER et al. [Theriogenology, (32) 515-525, (1989)] utilisent le dilueur de KENNEY et n'observent aucune différence de fertilité après utilisation du sperme conservé à 5°C ou à 20°C ; SQUIRES et al. [Proc. 11^{th} Int. Congress on Anim. Reprod. and AI, Dublin, Ireland, (3) 297,(1988)] utilisent le même dilueur et concluent à une meilleure fertilité du sperme conservé à 5°C, mais uniquement pour certains étalons.

De même la durée maximale de conservation a fait l'objet de rapports contradictoires. Certains auteurs [DOUGLAS-HAMILTON et al., Theriogenolgy, (22) 291-304, (1984)] ont utilisé les doses 6 heures à 24 heures après la récolte, et obtenu des gestations. D'autres, (SQUIRES et al. 1988, communication précitée) n'obtiennent par contre aucune gestation après conservation de la semence pendant 48 heures à 20°C. Une étude plus récente [HEISKANEN et al., Theriogenology (42) 1043-1051 (1994)] utilisant du sperme conservé 70 et 80 heures à 5°C dans le dilueur de Kenney supplémenté. en théophylline (10 mM) et Hepes (10 mM), donne des résultats de 77% et 57% de fertilité par cycle.

Le lait présente en effet des qualités qui ont fait préconiser son emploi en tant que dilueur : c'est un milieu tamponné qui contient des constituants potentiellement protecteurs des spermatozoïdes (lipides, protéines et lactose). Cependant, il s'agit d'un produit naturel et particulièrement complexe, contenant plus de 100 000 espèces moléculaires, dont la composition n'est pas totalement connue, et où la présence de constituants potentiellement néfastes pour les spermatozoïdes (c'est à dire spermicides ou diminuant la motilité des spermatozoïdes), a été soupçonnée [GARCIA et GRAHAM, Cryobiology (24) 446-454, (1987)]. En outre, du fait que le lait est un produit dont les composants ont une concentration variant sous l'effet de nombreux facteurs (stade de lactation, race, alimentation, etc.), les performances obtenues avec un dilueur à base de lait peuvent varier d'un lot à l'autre.

La présente Invention a pour but l'obtention de dilueurs à base de constituants du lait, de composition définie et reproductible, et permettant d'améliorer la conservation du sperme, et d'en contrôler les conditions.

Les Inventeurs ont découvert que, de façon surprenante, l'utilisation de fractions protéiques purifiées obtenues à partir du lait, en particulier le phosphocaséinate natif, et/ou la β-lactoglobuline, en remplacement du lait dans le milieu de dilution du sperme, permettait d'augmenter de façon très significative, par rapport aux dilueurs à base de lait connus dans l'art antérieur, aussi bien la durée de conservation du sperme, que le taux de fertilité obtenu après conservation. Les Inventeurs ont également constaté qu'en revanche, d'autres protéines laitières, comme l'α-lactalbumine, étaient toxiques pour les spermatozoïdes.

La présente Invention a pour objet l'utilisation d'une préparation purifiée de phosphocaséinate natif et/ou de β-lactoglobuline pour l'obtention d'un dilueur de sperme.

La présente Invention a en particulier pour objet un dilueur de sperme constitué par un -milieu de base formé de constituants convenant pour la dilution du sperme d'une espèce animale déterminée, et par une fraction purifiée de phosphocaséinate natif, et/ou une fraction purifiée de β-lactoglobuline.

Par « milieu de base comprenant les constituants convenant pour la dilution du sperme d'une espèce déterminée », on entend tout milieu comprenant les constituants habituellement utilisés dans les dilueurs de sperme utilisés pour ladite espèce, et dans lequel les constituants toxiques pour les spermatozoïdes, en particulier l'α-lactalbumine, sont absents ou ne sont présents que sous forme de traces, de manière à ce que ces constituants ne soient présents dans le dilueur conforme à l'invention qu'à une teneur au plus égale à 2 à 3% (en poids) de la teneur en β-lactoglobuline ou en phosphocaséinate natif et de préférence au plus égale à 0,5 à 1% (en poids) de la teneur en β-lactoglobuline ou en phosphocaséinate natif.

Un milieu de base convenant pour l'obtention d'un dilueur conforme à l'invention est par exemple constitué par une solution de sels minéraux et de glucides, tamponnée à un pH approprié. Avantageusement, il comprend également des additifs tels que des antibiotiques ou des antifongiques.

Selon un mode de réalisation préféré de la présente Invention, ledit dilueur de sperme comprend entre 1 et 100 g/l, de préférence entre 10 et 50 g/l de phosphocaséinate natif et/ou entre 10 et 200 g/l, de préférence entre 20 et 100 g/l de β-lactoglobuline.

La présente Invention a pour objet un procédé de préparation d'un dilueur de sperme, tel que défini ci-dessus, comprenant du phosphocaséinate natif, caractérisé en ce qu'il comprend au moins une étape au cours de laquelle on procède à la dissolution du phosphocaséinate natif dans un milieu aqueux, à une température comprise entre 30 et 80°C, de préférence entre 40 et 60°C.

La solution mère de phosphocaséinate natif obtenue de la sorte est ensuite mélangée avec un milieu de base tel que défini ci-dessus. Avantageusement, on la stérilise au préalable par traitement UHT. De préférence, pour éviter la déstabilisation du phosphocaséinate natif lors de la stérilisation, la solution mère comprend au moins 300, et de préférence 350 à 450 mg/l de calcium ionisé soluble ; la solubilisation des ions calcium peut notamment être obtenue par addition de citrate, par exemple du citrate disodique à une concentration de l'ordre de 2 à 5 g/l.

La présente Invention a également pour objet un dilueur de sperme susceptible d'être obtenu par le procédé conforme à l'invention.

La présente Invention a également pour objet des préparations du sperme en vue de l'insémination artificielle, caractérisée en ce que ledit sperme est dilué dans un dilueur conforme à l'invention.

La mise en oeuvre d'un dilueur de l'Invention améliore nettement la conservation du sperme, par rapport aux dilueurs à base de lait utilisées dans l'art antérieur.

Le sperme préparé en utilisant un dilueur conforme à l'Invention peut être conservé à des températures positives, comprises entre 4°C et 20°C, et avantageusement aux environs de 15°C. La température de conservation optimale dépend également de l'espèce considérée. Chez le cheval, par exemple, la mise en oeuvre d'un dilueur conforme à l'invention permet de conserver le sperme à 15°C en conditions aérobies, et d'obtenir une durée de conservation et un taux de fertilité supérieurs à ceux obtenus avec les dilueurs de l'art antérieur. Cette conservation à 15°C permet d'éviter l'effet de choc thermique intervenant habituellement lors du refroidissement du sperme.

Un dilueur conforme à l'invention peut être utilisé pour la conservation du sperme de différents mammifères, tels que par exemple les équins et les caprins, ainsi que pour la conservation du sperme d'autres animaux d'élevage, tels que les oiseaux.

La présente Invention sera mieux comprise à l'aide du complément de description qui va suivre, qui se réfère à des exemples non-limitatifs de préparation d'un dilueur conforme à l'Invention, et de son utilisation.

### EXEMPLE 1 - PREPARATION D'UN DILUEUR CONFORME A L'INVENTION

### 1) Fractionnement du lait

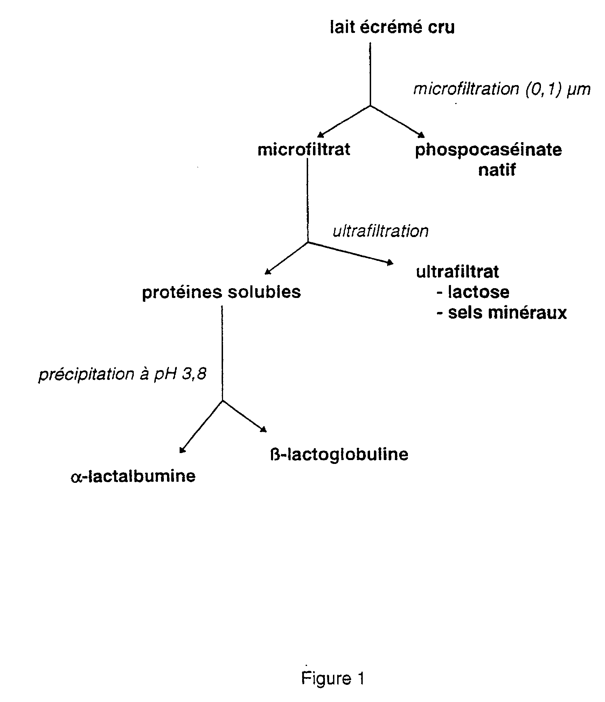

Le fractionnement des composants du lait écrémé cru est effectué par microfiltration sur membrane, [PIERRE et al. Lait (72) 461-474, (1992)] qui permet de séparer le microfiltrat (ou petit lait) du phosphocaséinate natif (caséines sous la forme micellaire qu'elles ont dans le lait). La β-lactoglobuline est obtenue à partir du microfiltrat, selon le protocole décrit par MAUBOIS et al. [Bull. IDF, 212, 154-159, (1987)] Les étapes de ce fractionnement sont schématisées sur la Figure 1 en annexe.

### 2) Milieu de base :

Le milieu de base utilisé est le milieu HGLL, dont la composition est la suivante :
0,140 g de CaCl₂
0,40 g de KCl
0,06 g de KH₂PO₄
0,2 g de MgSO₄, 7H₂O
8 g de NaCl
0,35 g de NaHCO₃
4,76 g d'Hepes
13,5 g de glucose
45 g de lactose
eau distillée q.s.p. 0,5 litres.

On ajoute à ce milieu de l'amphotéricine B, de la pénicilline, et de la gentamicine, pour parvenir à des concentrations finales dans le milieu de base respectives de 0,5 µg/µl, 500 UI/ml, et 100 µg/ml.

Ledit milieu de base est ensuite stérilisé par filtration sur une membrane dont le diamètre des pores est de 0,22 µm.

### 3) Préparation d'un dilueur à base de phosphocaséinate natif (dilueur PPCN).

On ajoute à 0,5 litres d'eau, à une température de 60°C, et sous agitation forte, 27 g de phosphocaséinate natif ; on poursuit l'agitation jusqu'à dissolution complète, et obtention d'une solution d'aspect laiteux. Cette solution est ensuite soumise à un traitement UHT (145°C, 2 secondes), puis mélangée à 0,5 litres de milieu de base obtenu comme décrit en 2) ci-dessus. Si nécessaire, le pH du mélange est ajusté à une valeur de 7,1. Le mélange est ensuite conditionné de façon aseptique.

### 4) Préparation d'un dilueur à base de β-lactoglobuline.

On ajoute à 0,5 litres de milieu de base obtenu comme décrit en 2) ci-dessus, 0,5 litres d'une solution de β-lactoglobuline à 80 g/l, préalablement stérilisée par filtration sur une membrane dont le diamètre des pores est de 0,22 µm. Si nécessaire, le pH du mélange est ajusté à une valeur de 7,1. Le mélange est conditionné de façon aseptique.

### 5) Préparation d'un dilueur PPCN supplémenté en citrate.

Pour effectuer une stérilisation plus poussée du dilueur, permettant une conservation optimale du sperme à 15°C, il est nécessaire de conserver la stabilité du phosphocaséinate natif (PPCN) lors du traitement thermique à haute température. Dans ce but, on maintient une concentration minimale en calcium ionisé soluble de l'ordre de 400 mg/l, par l'addition d'hydrogénocitrate disodique à la solution de phosphocaséinate natif.

La préparation du dilueur et sa stérilisation peuvent alors s'effectuer comme suit :
- une solution de phosphocaséinate natif (PPCN) (54g/l), préparée comme décrit ci-dessus, est additionnée de 2,65 g/1 d'hydrogénocitrate disodique (FLUKA 71 634), préalablement à sa stérilisation par traitement à ultra-haute température (140 à 144°C), pendant 3 à 4 secondes).
- une solution de milieu de base est préparée et stérilisée à froid par filtration sur membrane de 0,22 *µ*m, comme décrit en 2) ci-dessus.

Le mélange des 2 solutions stériles est ensuite réalisé comme décrit en 3) ci-dessus, et le dilueur final est conditionné stérilement sous forme de briques TETRAPAK®.

### EXEMPLE 2 : EFFET DES DIFFERENTES FRACTIONS DU LAIT SUR LA MOBILITE DES SPERMATOZOIDES IN VITRO

Différentes fractions du lait, à savoir le phosphocaséinate natif, et la β-lactoglobuline mentionnés ci-dessus, et également l'α-lactalbumine, et le résidu d'ultrafiltration ont été testées. Ces fractions sont additionnées au dilueur de base HGLL. A titre de témoins, on a utilisé du HGLL additionné de 1% de BSA (Serum Albumine Bovine, Sigma A7906), du lait UHT du commerce, et du dilueur INRA 82 [PALMER, Proc. 10^{th} Int. Congress on Anim. Reprod. and AI, Urbana-Champaign, IL USA, 3 :337, (1984)].

Dix étalons ont été utilisés au total, avec un minimum de 6 étalons (1 éjaculat par étalon) pour chaque test. Après la récolte, le sperme filtré sur de la gaze est dilué, pour obtenir une concentration de 20 × 10⁶ spermatozoides/ml, dans les différents milieux à tester, puis stocké en conditions aérobies à 15°C ou à 37°C (0,5 ml de sperme dilué dans des tubes de 5 ml), ou anaérobies à 4°C (5 ml de sperme dilué dans des tubes de 5 ml). Les durées de conservation ont varié entre 6 et 96 heures en fonction de la température et de la qualité des dilueurs utilisés afin que les observations soient réalisées à un moment où les dilueurs puissent être discriminés. Avant l'analyse, les échantillons sont réchauffés 10 minutes à 37°C. La qualité de la conservation du sperme *est* évaluée par analyse automatisée de la mobilité des spermatozoïdes (Hamilton Thorn Motility Analyser, Hamilton Thorn Research, Danvers, NIA, USA), en retenant comme critère le pourcentage de spermatozoïdes rapides c'est à dire ayant une vitesse supérieure à 30 µm/sec.

Les résultats sont illustrés par le Tableau I ci-après :

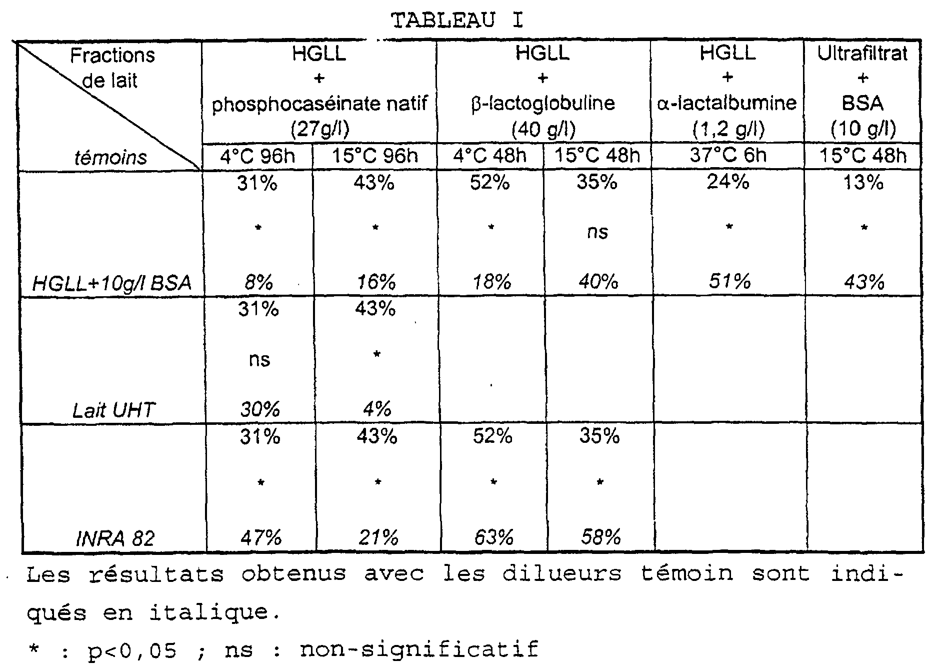

A 15°C après 96 heures de conservation, le pourcentage de spermatozoïdes rapides le plus élevé est obtenu avec le dilueur HGLL + phosphocaséinate natif. Par contre à 4°C, les résultats obtenus avec ce di-lueur sont identiques à ceux obtenus avec le lait, et inférieurs à ceux obtenus avec l'INRA 82.

La β-lactoglobuline permet d'obtenir des résultats significativement supérieurs à ceux obtenus avec la BSA à 4°C ; par contre à 15°C ces 2 protéines apportent une protection identique. L'α-lactalbumine et l'ultrafiltrat sont 2 fractions toxiques pour les spermatozoïdes.

L'effet éventuel de la supplémentation par l'hydrogénocitrate disodique a également été testé.

La semence provenant de 20 éjaculats a été conservée pendant 7 jours à 15°C, dans le dilueur HGLL-PPCN additionné ou non d'hydrogénocitrate disodique, obtenu comme décrit à l'exemple 1 ci-dessus. Le pourcentage de spermatozoïdes rapides a été évalué comme décrit ci-dessus. Il est de 56,58 % pour le HGLL-PPCN supplémenté en hydrogénocitrate disodique, et de 55,49 % pour le HGLL-PPCN seul.

Ces résultats montrent que l'addition d'hydrogénocitrate disodique n'induit aucun effet significatif sur la mobilité des spermatozoides.

### EXEMPLE 3 : EFFET D'UN DILUEUR CONFORME A L'INVENTION COMPRENANT DU PHOSPHOCASEINATE NATIF, SUR LA FERTILITE IN VIVO

### 1) Sperme conservé pendant 24 heures

Quatre expériences utilisant du sperme conservé 24 heures ont été réalisées. Dans chacune de ces 4 expériences deux types de conditions de conservation du sperme ont été comparées :
- dans l'INRA 82 ou le dilueur de Kenney à 4°C, en conditions anaérobies dans des seringues de 20 ml ne contenant que 10 ml de sperme dilué (piston tiré à moitié).
- dans le dilueur HGLL + phosphocaséinate natif conforme à l'invention, à 15°C en conditions aérobies dans des seringues de 20 ml contenant 10 ml de sperme dilué et 10 ml d'air (piston tiré complètement).

La semence d'étalon est diluée à une concentration de 20 × 10⁶ spermatozoïdes par ml. Les races des étalons, leur nombre, le nombre de cycles ont varié selon les expériences. Les conditions expérimentales sont résumées dans le tableau II ci-après.

**TABLEAU II**

| N° d'expérience | 1 | 2 | 3 | 4 |
|---|---|---|---|---|
| Animaux | Poneys | Chevaux de selle et lourds | | Chevaux de selle |
| Nombre d'étalons | 2 | 3 | 4 | 4 |
| Nombre de cycles | 84 | 90 | 125 | 62 |
| Dilueur 4°C | INRA 82 | Diiueur de KENNEY | Dilueur de KENNEY | Dilueur de KENNEY |

Les ponettes et les juments ont été échographiées chaque jour à partir du premier jour des chaleurs et réparties au hasard dans les 2 lots. Dans l'expérience n°1, les ponettes ont été inséminées une première fois quand le follicule dominant a atteint 33 mm. L'ovulation a été induite 24 heures après par une injection intraveineuse de 25 mg de C.E.G (crude equine gonadotrophin ou extrait hypophysaire équin total) et une seconde insémination a été réalisée 48 heures après la première, si l'ovulation n'était pas survenue. Dans les expériences n° 2 à 4, les conditions ont été plus proches des conditions d'élevage : les juments ont été inséminées à partir du jour où le follicule dominant a atteint 33 mm, toutes les 48 heures jusqu'à ovulation.

Le tableau III ci-dessous regroupe les résultats de ces 4 expériences, exprimés en pourcentage de femelles chez lesquelles le diagnostic de gestation est positif au 14^{ème} jour après l'ovulation :

**TABLEAU III**

| N° d'expérience | 1 | 2 | 3 | 4 | Total |
|---|---|---|---|---|---|
| 4°C | 36% n = 41 | 39%n=44 | 34%n=64 | 58% n = 31 | 41% n = 171 |
| 15°C | 60% n = 43 | 50% n = 46 | 47% n = 61 | 87% n = 31 | 57% n = 176 |

Dans chaque expérience les doses conservées à 15°C avec le dilueur conforme à l'invention ont permis d'obtenir une fertilité par cycle supérieure à celle obtenue avec les doses conservées à 4°C.

### 2) Sperme conservé pendant 72 heures

Une expérience utilisant du sperme conservé 72 heures avant l'insémination a également été réalisée. Toutes les doses ont été préparées dans le dilueur HGLL + phosphocaséinate natif conforme à l'invention, et ont été utilisées soit pour insémination immédiate, soit après conservation à 15°C avec agitation constante.

Les ponettes ont été échographiées chaque jour à partir du premier jour des chaleurs et réparties au hasard dans les 2 lots. Cent deux cycles de ponettes ont été utilisés au total. Dans la première partie de l'expérience, toutes les ponettes ont été inséminées une seule fois, environ 7 heures avant l'ovulation induite par une injection intraveineuse de 25 mg de C.E.G. Dans la seconde partie, les ponettes du lot "inséminations différées" ont été inséminées 2 fois : la première fois environ 12 heures avant l'ovulation induite et la seconde fois environ 4 heures après l'ovulation.

Les résultats (en pourcentage de femelles chez lesquelles le diagnostic de gestation est positif au 14^{ème} jour après l'ovulation) sont illustrés par le tableau IV ci dessous :

**TABLEAU IV**

| | IA immédiate | IA 72 heures |
|---|---|---|
| Première partie | 65% n = 17 | 50% n = 20 |
| Seconde partie | 70% n = 33 | 47% n = 32 |
| Total | 68 % n = 50 | 48% n = 52 |

Les résultats obtenus pour les 2 parties de l'expérience sont identiques, et ne dépendent pas du le nombre d'inséminations, ou de la présence d'une seconde insémination post-ovulation très proche du moment de l'ovulation. Les pourcentages de fertilité par cycle sont de 48% obtenus après inséminations différées de 72 heures, et de 68% obtenus après inséminations immédiates.

Ces résultats montrent que la fertilité obtenue après 72 heures de conservation du sperme à 15°C dans le dilueur HGLL + phosphocaséinate natif est d'une qualité qui permet son utilisation dans les élevages.

### EXEMPLE 4 : EFFET D'UN DILUEUR CONFORME A L'INVENTION COMPRENANT DU PHOSPHOCASEINATE NATIF, SUR LA MOBILITE IN VIVO DE SPERME CAPRIN.

Dans l'espèce caprine, la conservation du sperme s'effectue classiquement à 4°C, après élimination du plasma séminal et dilution dans du lait (lait en poudre reconstitué et chauffé à 92°C pendant 10 minutes). La semence diluée est ensuite conditionnée en aliquotes de 2 ml placées dans des tubes de 5 ml.

En utilisant ce même protocole, les Inventeurs ont comparé la mobilité des spermatozoïdes, après conservation à 4 ou à 15°C :
- soit dans un dilueur conforme à l'invention, obtenu par l'addition de phosphocaséinate natif (27 g/l) à un milieu de base dénommé KGB, qui est constitué par du milieu BES-KOH (DUNNER, Anim. Prod., 56, 387-391, (1993) ; DUNNER et IMPASTO, Small Ruminant Research, 11, 57-64, (1993)] supplémenté avec 2mg/ml de glucose,
- soit dans du lait.

Deux types de conditionnement ont également été testés : le conditionnement aérobie classique (2 ml de semence diluée, dans des tubes de 5 ml) mentionné ci-dessus, ou bien un conditionnement anaérobie sous forme de « paillettes » de 0,5 ml totalement remplies de semence diluée.

Les résultats de mobilité obtenus à partir de 10 pools d'éjaculats sont représentés sur dans les tableaux V et VI ci-dessous. Le tableau V représente le pourcentage de spermatozoïdes rapides (analyse automatisée), et le tableau VI représente les résultats d'une évaluation de la mobilité par observation au microscope et appréciation du pourcentage de spermatozoïdes mobiles et de la qualité du mouvement, selon les critères définis par [BISHOP et al., J. Agriculture Science, 44, 227, (1954)] ; les résultats sont notés sur une échelle de 0 à 5.

**TABLEAU V**

| | | | | |
|---|---|---|---|---|
| Dilueur | Lait | Lait | KGB + PPCN | KGB + PPCN |
| Conditionnement | Paillettes | Tubes | Paillettes | Tubes |
| 4°C | 18% | 8% | 40% | 32% |
| 15°C | 0% | 0% | 0% | 6% |

**TABLEAU VI**

| | | | | |
|---|---|---|---|---|
| Dilueur | Lait | Lait | KGB + PPCN | KGB + PPCN |
| Conditionnement | Paillettes | Tubes | Paillettes | Tubes |
| 4°C | 1 | 0,4 | 2,4 | 3,1 |
| 15°C | 0 | 0 | 0 | 0,6 |

Ces résultats montrent que l'utilisation d'un dilueur conforme à l'invention améliore très significativement la survie à 4°C des spermatozoides caprins.

## Revendications

1. Dilueur de sperme, constitué par-un milieu de base formé de constituants convenant pour la dilution du sperme d'une espèce animale déterminée, et par une fraction purifiée de phosphocaséinate natif, et/ou une fraction purifiée de β-lactoglobuline.

2. Dilueur de sperme selon la revendication 1 caractérisé en ce qu'il comprend entre 1 et 100 g/l de phosphocaséinate natif et/ou entre 10 et 200 g/l de β-lactoglobuline.

3. Dilueur de sperme selon la revendication 2 caractérisé en ce qu'il comprend entre 10 et 50 g/l de phosphocaséinate natif et/ou entre 20 et 100 g/l de β-lactoglobuline.

4. Procédé de préparation d'un dilueur de sperme selon une quelconque des revendications 1 à 3, comprenant du phosphocaséinate natif, caractérisé en ce qu'il comprend au moins une étape au cours de laquelle on prépare une solution de phosphocaséinate natif, en procédant à la dissolution dudit phosphocaséinate natif dans un milieu aqueux, à une température comprise entre 30 et 80°C, de préférence entre 40 et 60°C.

5. Procédé selon la revendication 4 caractérisé en ce que ladite solution de phosphocaséinate natif est stérilisée par traitement UHT.

6. Préparation de sperme en vue de l'insémination artificielle, caractérisée en ce que ledit sperme est dilué dans un dilueur selon une quelconque des revendications 1 à 3.

7. Utilisation d'une fraction purifiée de phosphocaséinate natif, et/ou d'une fraction purifiée de β-lactoglobuline, pour l'obtention d'un dilueur de sperme.

8. Utilisation d'un dilueur de sperme selon une quelconque des revendications 1 à 3, pour la conservation de sperme de mammifère en vue de l'insémination artificielle.

9. Utilisation selon la revendication 8, caractérisée en ce que ledit mammifère appartient à l'espèce équine.

10. Utilisation selon la revendication 8, caractérisé en ce que ledit mammifère appartient à l'espèce caprine.

11. Utilisation selon une quelconque des revendications 8 ou 9, caractérisée en ce que ladite conservation est effectuée à une température de 15°C en conditions aérobies.

## Patentansprüche

1. Verdünnungsmittel für Sperma bestehend aus einem Grundmedium, gebilde aus zur Verdünnung von Sperma einer bestimmten Tierart geeigneten Be standteilen, und einer gereinigten Fraktion von nativem Phosphocaseina und/oder einer gereinigten Fraktion von β-Lactoglobulin.

2. Verdünnungsmittel für Sperma nach Anspruch 1, dadurch gekennzeichnet dass es 1 bis 100 g/l natives Phosphocaseinat und/oder 10 bis 200 g/l β Lactoglobulin umfasst.

3. Verdünnungsmittel für Sperma nach Anspruch 2, dadurch gekennzeichnet dass es 10 bis 50 g/l natives Phosphocaseinat und/oder 20 bis 100 g/l β Lactoglobulin umfasst.

4. Verfahren zur Herstellung eines Verdünnungsmittels für Sperma nach einer der Ansprüche 1 bis 3, umfassend natives Phosphocaseinat, dadurch ge kennzeichnet, dass es mindestens eine Stufe umfasst, im Verlauf derer mai eine Lösung aus nativem Phosphocaseinat herstellt, indem man das native Phosphocaseinat in einem wässrigen Medium bei einer Temperatur zwischen 30 und 80 °C, bevorzugt zwischen 40 und 60 °C, auflöst.

5. Verfahren nach Anspruch 4, dadurch gekennzeichnet, dass die Lösung au nativem Phosphocaseinat durch UHT-Behandlung sterilisiert wird.

6. Verfahren zur Herstellung von Sperma im Hinblick auf eine künstliche Be fruchtung, dadurch gekennzeichnet, dass das Sperma mit einem Verdün nungsmittel nach einem der Ansprüche 1 bis 3 verdünnt wird.

7. Verwendung einer gereinigten Fraktion von nativem Phosphocaseina und/oder einer gereinigten Fraktion von β-Lactoglobulin zum Erhalt eines Vei dünnungsmittels für Sperma.

8. Verwendung eines Verdünnungsmittels für Sperma nach einem der Ansprüche 1 bis 3 zur Konservierung von Säugersperma im Hinblick auf eine künstliche Befruchtung.

9. Verwendung nach Anspruch 8, dadurch gekennzeichnet, dass der Säuger der Art Pferd angehört.

10. Verwendung nach Anspruch 8, dadurch gekennzeichnet, dass der Säuger der Art Ziege angehört.

11. Verwendung nach einem der Ansprüche 8 oder 9, dadurch gekennzeichnet, dass die Konservierung bei einer Temperatur von 15°C unter aeroben Bedingungen durchgeführt wird.

## Claims

1. Sperm diluent composed of a base medium formed from constituents suitable for diluting the sperm of a defined species of animal and a purified fraction of native phosphocaseinate and/or a purified fraction of β-lactoglobulin.

2. Sperm diluent according to Claim 1, characterized in that it comprises between 1 and 100 g/l of native phosphocaseinate and/or between 10 and 200 g/l of β-lactoglobulin.

3. Sperm diluent according to Claim 2, characterized in that it comprises between 10 and 50 g/l of native phosphocaseinate and/or between 20 and 100 g/l of β-lactoglobulin.

4. Method for preparation of a sperm diluent according to any of the Claims 1 to 3, comprising native phosphocaseinate, characterized in that it comprises at least one stage during which a solution of native phosphocaseinate is prepared by dissolving the said native phosphocaseinate in an aqueous medium at a temperature between 30 and 80°C, preferably between 40 and 60°C.

5. Method according to Claim 4, characterized in that the said solution of native phosphocaseinate is sterilised by UHT treatment.

6. Sperm preparation intended for artificial insemination, characterized in that the said sperm is diluted in a diluent according to any of the Claims 1 to 3.

7. Use of a purified fraction of native phosphocaseinate and/or a purified fraction of β-lactoglobulin in manufacture of a sperm diluent.

8. Use of a sperm diluent according to any of the Claims 1 to 3 to preserve mammalian sperm intended for artificial insemination.

9. Use according to Claim 8, characterized in that the said mammal belongs to an equine species.

10. Use according to Claim 8, characterized in that the said mammal belongs to a caprine species.

11. Use according to any of the claims 8 or 9, characterized in that the said preservation is carried out at a temperature of 15°C in aerobic conditions.
